# DEMANDE DE BREVET EUROPEEN

(11) **EP 2 305 212 A1**
(43) Date de publication de la demande: **06.04.2011**
(21) Numéro de dépôt: 10166501.6
(22) Date de dépôt: 18.06.2010
(51) Int. Cl.: A61K 8/97, A61K 8/99, A61Q 19/02, A61Q 19/06, A61Q 19/08, A61Q 19/00

(54) **Composition contenant un extrait de bactérie filamenteuse et procédé de traitement cosmétique des signes du vieillissement**

(30) Priorité: 22.06.2009 FR 0954220
(71) Demandeur: L'Oréal, 75008 Paris (FR)
(72) Inventeur: Amar, Camille, 92120, Montrouge (FR); Cheilian, Stéphanie, 75003, Paris (FR); Saussay, Sophie, 95470, Survilliers (FR); Mahe, Yann, 91700, Sainte Genevieve Des Bois (FR)
(74) Mandataire: Allab, Myriam

(57) **Abrégé**

L'invention concerne une composition qui contient dans un milieu physiologiquement acceptable l'association d'au moins un extrait d'une bactérie filamenteuse non photosynthétique et non fructifiante et au moins un extrait de la plante Cassia alata. Elle concerne également une association d'un extrait de Vitreoscilla filiformis et de Cassia alata, ainsi que des procédés de traitement cosmétique les mettant en oeuvre.

## Description

La présente invention concerne de nouvelles compositions, notamment à usage cosmétique ou dermatologique, et leur utilisation dans le domaine de l'hydratation, de la lutte contre les signes du vieillissement, de l'amincissement ou pour éclaircir la couleur de la peau.

La peau constitue une barrière contre les agressions extérieures, notamment chimiques, mécaniques ou infectieuses, et à ce titre un certain nombre de réactions de défense contre les facteurs environnementaux (climat, rayons ultraviolets, tabac, ...) et/ou les xénobiotiques, comme par exemple les micro-organismes, se produisent à son niveau. La peau est constituée de deux compartiments, l'un superficiel, l'épiderme, et un plus profond, le derme, qui interagissent. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures appelé "fonction barrière".

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau. L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline et enfin la couche cornée (ou *stratum corneum*)*,* constituée d'un ensemble de couches de kératinocytes au stade terminal de leur différenciation appelés cornéocytes.

La Transglutaminase K (ou TGK) est une enzyme appartenant à la famille des transpeptidases. C'est une enzyme calcium-dépendante qui catalyse la formation des ponts isopeptidiques ε(γ-glutamyl) lysine entre protéines épidermiques et favorise donc la formation des enveloppes cornées.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses. Dans une peau normale, c'est à dire non pathologique ni cicatricielle, le fibroblaste est à l'état quiescent, c'est à dire non prolifératif, peu actif d'un point de vue métabolique et non mobile.

Ce sont les fibres de collagène qui assurent la solidité du derme. Les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.

Les fibres de collagènes sont constamment renouvelées mais ce renouvellement diminue avec l'âge ce qui entraîne un amincissement du derme. Cet amincissement du derme est également dû à des causes pathologiques comme par exemple l'hypersécrétion d'hormones corticoïdes, certaines pathologies ou encore des carences vitaminiques (cas de la vitamine C dans le scorbut). Il est également admis que des facteurs extrinsèques comme les rayons ultraviolets, le tabac ou certains traitements (Glucocorticoïdes, vitamine D et dérivés par exemple) ont également un effet sur la peau et sur son taux de collagène.

Cependant, divers facteurs entraînent la dégradation du collagène, avec toutes les conséquences que l'on peut envisager sur la structure et/ou la fermeté de la peau et/ou des muqueuses.

Bien que très résistantes, les fibres de collagène sont sensibles à certaines enzymes appelées collagénases. Une dégradation des fibres de collagène entraîne l'apparence de peau molle et ridée que l'être humain, préférant l'apparence d'une peau lisse et tendue, cherche depuis toujours à combattre.

Les collagénases font partie d'une famille d'enzymes appelées métalloprotéinases (MMPs) qui sont elles-mêmes les membres d'une famille d'enzymes protéolytiques (endoprotéases) qui possèdent un atome de zinc coordonné à 3 résidus cystéine et une méthionine dans leur site actif et qui dégradent les composants macromoléculaires de la matrice extracellulaire et des lames basales à pH neutre (collagène, élastine, etc....). Très largement répandues dans le monde vivant, ces enzymes sont présentes, mais faiblement exprimées, dans des situations physiologiques normales comme la croissance des organes et le renouvellement des tissus.

Leur surexpression chez l'homme et leur activation sont cependant liées à de nombreux processus, qui impliquent la destruction et le remodelage de la matrice. Cela entraîne soit une résorption non contrôlée de la matrice extracellulaire, soit inversement l'installation d'un état de fibrose.

Les métalloprotéinases sont produites et sécrétées sous une forme zymogène inactive (pro-enzyme). Ces formes zymogènes sont ensuite activées dans l'environnement extracellulaire par l'élimination d'une région propeptidique. Les membres de cette famille peuvent s'activer les uns les autres.

La régulation de l'activité des MMPs se produit ainsi au niveau de l'expression des gènes (transcription et traduction), au niveau de l'activation de la forme zymogène, ou au niveau du contrôle local des formes actives.

Les principaux régulateurs de l'activité des MMPs sont les inhibiteurs tissulaires des métalloprotéinases ou TIMPs (tissue inhibitors of metalloproteinases).

La famille des métalloprotéinases est constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa) ), les stromélysines (MMP-3) dont le large spectre d'activité s'adresse aux protéines de la matrice extracellulaire telles que les glycoprotéines (fibronectine, laminine), les protéoglycannes, etc., ou encore les métalloprotéinases membranaires.

L'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit.

Par ailleurs, les rondeurs et/ou surcharges pondérales sont liées au dysfonctionnement de certaines cellules de l'hypoderme, appelées adipocytes, qui contiennent des quantités variables de graisses stockées sous la forme de triglycérides. Ces triglycérides sont synthétisés in vivo par les adipocytes eux-mêmes, selon des réactions de type enzymatique (lipogenèse), à partir des acides gras libres et du glucose contenus dans l'organisme et apportés à celui-ci par l'intermédiaire de certains aliments. La transformation du glucose conduit soit à la formation de glycérol, soit à la formation d'acides gras libres par l'intermédiaire d'une enzyme spécifique, l'acétylCoA-carboxylase. Or, parallèlement, les triglycérides ainsi formés, puis stockés, dans les cellules adipocytes peuvent également se dégrader (lipolyse), toujours sous l'action d'enzymes spécifiques (triglycérides lipases) contenues dans ces mêmes cellules, en libérant cette fois des acides gras d'une part et du glycérol et/ou des mono- et/ou des di-esters du glycérol d'autre part. Les acides gras ainsi relargués peuvent alors soit diffuser dans l'organisme pour y être consommés ou transformés de différentes façons, soit être recaptés (aussitôt ou un peu plus tard) par les adipocytes pour générer à nouveau des triglycérides par lipogenèse.

Si, pour des raisons diverses (nourriture trop riche, inactivité, variation du métabolisme, vieillissement et autres), un déséquilibre substantiel s'installe dans l'organisme entre la lipogenèse (formation de triglycérides par réaction enzymatique entre des acides gras et le glycérol) et la lipolyse (décomposition enzymatique de triglycérides en acides gras et glycérol), c'est-à-dire plus précisément si les quantités de graisses formées par lipogenèse deviennent notablement et constamment supérieures à celles qui sont éliminées par lipolyse, il se produit alors dans les adipocytes une accumulation de triglycérides qui, si elle devient excessive, peut se traduire progressivement par une déformation de la peau provoquée par l'épaississement de l'hypoderme dans lequel se trouvent les adipocytes. La surface de la peau devient irrégulière ("peau d'orange") et de consistance plus ou moins flasque ou gélatineuse, donnant finalement à la silhouette un aspect général disgracieux pouvant évoluer entre la simple surcharge locale (lipodismorphie), en passant par l'embonpoint certain, et enfin la réelle obésité.

Or, compte tenu notamment du profond inconfort tant physique qu'esthétique, et parfois psychologique, qu'elle occasionne auprès des individus qui en sont atteints, en particulier chez les femmes, l'adiposité constitue de nos jours une affection de moins en moins bien supportée ou acceptée.

Il existe donc un besoin de trouver des actifs améliorant l'homéostasie de la peau et de ses annexes, en maintenant un bon équilibre de ses différentes fonctions et en luttant contre les dégradations qui se produisent de manière physiologique au cours du temps.

Ces buts et d'autres sont atteints par une association d'actifs dont l'effet est ainsi amélioré.

C'est pour quoi la présente invention a pour objet une composition contenant dans un milieu physiologiquement acceptable l'association d'au moins un extrait d'une bactérie filamenteuse non photosynthétique et non fructifiante et au moins un extrait de la plante Cassia alata.

L'extrait bactérien utilisé selon l'invention est préparé suivant un procédé comprenant la culture d'au moins une bactérie filamenteuse non photosynthétique et non fructifiante.

Les bactéries utilisées sont des bactéries filamenteuses non photosynthétiques qui comprennent notamment les bactéries appartenant à l'ordre des Beggiatoales, et plus particulièrement les bactéries appartenant aux genres *Beggiatoa, Vitreoscilla, Flexithrix* ou *Leucothrix.*

Pour la mise en oeuvre de l'invention, on préfère les bactéries appartenant au genre Vitreoscilla, en particulier pour les bactéries de l'espèce *Vitreoscilla filiformis.*

Ces bactéries dont plusieurs ont déjà été décrites ont généralement un habitat aquatique, et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)

De préférence, la bactérie est celle correspondant à la souche déposée à l'ATCC sous le n°15551.

L'effet immunomodulateur et apaisant dudit extrait bactérien et des compositions cosmétiques pour cils ou cheveux comprenant un extrait de bactérie filamenteuse non photosynthétique non fructifiante associé à un filtre UV organique ont été décrites (WO02/056858), ou des compositions pour cheveux comprenant ledit extrait associé à un produit à effet irritant (EP0761204). La demande EP0876813 (L'OREAL) concerne une composition comprenant une quantité efficace de milieu de culture d'au moins une bactérie filamenteuse non photosynthétique.

Pour préparer l'extrait de bactérie selon l'invention, on peut cultiver lesdites bactéries selon les méthodes connues de l'homme du métier, ou se reporter en particulier à la description de la demande de brevet WO-A-93-00741. On obtient un extrait cellulaire dont on peut séparer le surnageant par exemple par filtration et centrifugation. L'extrait peut être utilisé sous forme aqueuse ou sous forme lyophilisée.

De façon connue, le procédé de préparation de l'extrait bactérien comprend au moins une étape dans laquelle on récupère les bactéries à la fin de la culture, en particulier en les séparant du milieu de culture.

Après culture des bactéries, on peut isoler la biomasse par diverses méthodes connues, par exemple par filtration, par coagulation avec un alcool (éthanol, isopropanol, isobutanol), par séchage sur cylindre à précouche (amidon, diatomées...) raclée, ou par lyophilisation. Une concentration préalable, par exemple à 80°C sous pression réduite, améliore cette séparation.

La biomasse peut être utilisée vivante ou bien être traitée par différents procédés. On peut procéder à une opération de rupture des enveloppes, par exemple par l'action des ultrasons. On peut en outre préparer des extraits à l'aide d'un alcool tel que l'éthanol ou le propanol.

On peut également préparer des extraits lipopolysaccharidiques selon les méthodes connues, voir par exemple Noris et Ribbons, Methods in Microbiology, Vol. 5B, Academic Press (1971). La méthode généralement utilisée est la méthode bien connue dite de Westphal (ou une méthode apparentée), qui consiste à faire l'extraction avec des mélanges phénol-eau à 65°C. On soumet ensuite l'extrait à une dialyse pour éliminer le phénol.

L'extrait bactérien utile selon l'invention peut encore résulter de la mise en oeuvre du procédé suivant : (i) on cultive au moins une bactérie appartenant à l'ordre des Beggiatoales sur un milieu comprenant un ose comme source principale de carbone, puis (ii) après fermentation, on sépare les bactéries du milieu de culture, pour récupérer ladite masse des bactéries.

Les bactéries récupérées à l'issue de l'étape de fermentation peuvent notamment être soumises à un traitement de stabilisation et/ou d'extraction. C'est l'extrait de bactéries filamenteuses ainsi obtenu qui sera généralement utilisé dans ou pour la préparation de compositions cosmétiques ou dermatologiques. De manière connue en soi, l'extrait pourra ainsi être stérilisé en particulier par filtration ou par autoclavage.

Par extrait de bactéries filamenteuses non photosynthétiques, on entend aussi bien le surnageant de culture desdites bactéries, la biomasse obtenue après culture desdites bactéries ou encore les extraits de la biomasse obtenus par traitement de cette biomasse.

Pour préparer l'extrait selon l'invention, on peut cultiver lesdites bactéries selon le procédé selon l'invention, puis les séparer de la biomasse obtenue, par exemple par filtration, centrifugation, coagulation et/ou lyophilisation.

Ainsi, après culture, les bactéries sont concentrées par centrifugation. La biomasse obtenue est autoclavée. Cette biomasse peut être lyophilisée pour constituer ce que l'on appelle l'extrait lyophilisé. Toute méthode de lyophilisation connue de l'homme du métier est utilisable pour préparer cet extrait.

La fraction surnageante de cette biomasse peut également être filtrée dans un récipient stérile pour éliminer les particules en suspension. Cette fraction surnageante peut également être transvasée stérilement dans un récipient stérile. Selon un mode de réalisation particulier de l'invention, la fraction surnageante ainsi obtenue est utilisée à titre d'actif cosmétique ou dermatologique.

Avantageusement, l'extrait utile dans les compositions selon l'invention est constitué des bactéries séparées du milieu de culture, par exemple par centrifugation; il peut ensuite être soumis à des traitements, notamment de congélation et de stérilisation.

L'extrait bactérien utile selon l'invention peut être formulé dans un support approprié à raison d'au moins 20% en poids par rapport au poids total de la composition, en particulier à raison de 0,001 à 20% en poids par rapport au poids total de la composition et plus particulièrement à raison de 0,01 à 10% en poids par rapport au poids total de la composition. Selon un mode de réalisation avantageux, l'extrait de bactérie filamenteuse non photosynthétique est présent à une concentration de 0,1 à 10% en poids par rapport au poids total de la composition.

Cassia alata ou senna alata est connu sous le nom de dartrier ou casse ailé. C'est une plante arborescente de la famille des Caesalpiniaceae ou des Fabaceae. Il appartient au genre cassia qui compte plus de 600 espèces. C'est un petit arbuste reconnaissable à ses fleurs jaunes en épis dressés. Il est proposé pour des propriétés purgatives, antibiotiques ou antiparasitaires.

Avantageusement pour la mise en oeuvre de l'invention, on utilise un extrait de feuille de Cassia alata, notamment un extrait de Senna alata (L.) Roxb sélectionné pour sa teneur en polyphenols, et plus particulièrement en flavonoïdes, et plus particulièrement encore en Kaempferol-3-O-Sophoroside.

L'extrait de Cassia alata pourra être présent dans les compositions selon l'invention à une concentration de 10⁻⁵ à 20 %, de préférence de 10⁻³ à 10 %, plus préférentiellement encore de 0,01 à 5 %, en poids par rapport au poids total de la composition. Selon un mode de réalisation avantageux, l'extrait de Cassia alata est présent à une concentration de 0,001% à 1%, de préférence de 0.025% à 0,5 % en poids par rapport au poids total de la composition.

En effet il a été trouvé dans le cadre de la présente invention que des compositions renfermant une association d'au moins un extrait de bactérie filamenteuse et d'au moins un extrait de Cassia alata présentent des propriétés améliorées d'augmentation de la synthèse duTIMP1 et /ou d'augmentation de la synthèse de transglutaminase K (TGK) en particulier de la TGK1 par les cellules. L'invention concerne donc des compositions présentant une activité synergique sur ces 2 activités, qui sont particulièrement avantageuses notamment pour améliorer l'hydratation et/ou la fermeté de la peau. En particulier, la synthèse de TIMP-1 en présence des 2 actifs est supérieure à la somme de celle obtenue avec chaque actif séparément dès 6 heures après la mise en contact avec des cellules cutanées.

Les associations d'au moins un extrait de bactérie filamenteuse non photosynthétique et d'un extrait de Cassia alata, ou les compositions les contenant telles que définies dans ce qui précède auront ainsi une activité de stimulation de la différenciation épidermique et de protection de la matrice extracellulaire vis-à-vis de la dégradation par la MMP1.

C'est pourquoi la présente invention a pour objet une composition contenant l'association d'au moins un extrait d'une bactérie filamenteuse non photosynthétique et non fructifiante et au moins un extrait de la plante Cassia alata telle que définie précédemment, ladite composition étant une composition cosmétique et/ou dermatologique, contenant en outre des excipients adaptés à une application topique sur la peau et/ou les muqueuses et/ou le cuir chevelu.

Les compositions selon l'invention sont notamment des compositions à activité hydratante, anti-âge, blanchissante, amincissante ou des compositions solaires.

Les compositions selon l'invention peuvent contenir des actifs cosmétiques complémentaires, et notamment au moins un autre actif choisi parmi les agents anti-âge, les dépigmentants, les filtres U.V. et les agents hydratants.

Par agents anti-âge on entend notamment les agents anti-rides, les agents antioxydants, les agents dermodécontractants ou agents dermorelaxants, les agents anti-glycation, les agents stimulant la synthèse des macromolécules dermiques et/ou épidermiques et/ou empêchant leur dégradation et les agents stimulant la prolifération des fibroblastes ou des kératinocytes et/ou la différenciation des kératinocytes.

Selon un autre aspect de l'invention, la composition contenant l'association est destinée à une administration par voie orale, et contient des excipients adaptés à cette voie.

L'invention a également pour objet une association synergique d'au moins un extrait de Vitreoscilla filiformis et d'au moins un extrait de Cassia alata, présentant une synergie d'activité sur l'augmentation de la synthèse de la transglutaminase K et/ou l'augmentation de synthèse de la TIMP1 par les cellules de la peau.

### Actifs cosmétiques complémentaires

Selon un mode de réalisation avantageux, l'association selon l'invention peut être associée à un ou plusieurs actifs cosmétiques complémentaires.

Ces actifs pourront être choisis parmi les agents anti-rides les filtres UV, les vitamines en particulier B3, B8, B12 et B9, les agents hydratants, les agents desquamants, les actifs anti-âges, les agents améliorant la fonction barrière, les agents dépigmentants, les agents antioxydants, les agents dermodécontractants ou agents dermorelaxants, les agents anti-glycation, les agents stimulant la synthèse des macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules et les agents apaisants.

Selon une variante particulière de l'invention, l'association selon l'invention est mise en oeuvre conjointement avec au moins un actif choisi parmi les actifs anti-rides les filtres UV, les agents antioxydants, les actifs stimulant la synthèse des macromoléculaires dermiques et/ou épidermiques, les agents dermodécontractants et leurs mélanges.

Ces actifs complémentaires pourront être présents dans la composition en une teneur allant de 0,001 à 20 % en poids, de préférence de 0,01 à 10 % en poids, et plus préférentiellement de 0,01 à 5 % en poids par rapport au poids total de la composition.

### Agents anti-rides

Des exemples d'actifs anti-rides additionnels utilisables selon l'invention sont : l'acide ascorbique et ses dérivés, tels que l'ascorbyl phosphate de magnésium et le glucoside d'ascorbyle ; le tocophérol et ses dérivés, tels que l'acétate de tocophéryle ; l'acide nicotinique et ses précurseurs, tels que la nicotinamide ; l'ubiquinone ; le glutathion et ses précurseurs, tels que l'acide L-2-oxothiazolidine-4-carboxylique ; les composés C-glycoside et leurs dérivés, tels que décrits notamment ci-après ; les extraits de plantes et notamment les extraits de criste marine et de feuille d'olivier, ainsi que les protéines végétales et leurs hydrolysats, tels que les hydrolysats de protéines de riz ou de soja ; les extraits d'algues et en particulier de laminaires ; les extraits bactériens ; les sapogénines, telles que la diosgénine et les extraits de Dioscorées, en particulier de Wild Yam, en contenant ; les α-hydroxyacides ; les β-hydroxyacides, tels que l'acide salicylique et l'acide n-octanoyl-5-salicylique ; les oligopeptides et pseudodipeptides et leurs dérivés acylés, en particulier l'acide {2-[acetyl-(3-trifluoromethyl-phenyl)-amino]-3-methylbutyrylamino} acétique et les lipopeptides commercialisés par la société SEDERMA sous les dénominations commerciales Matrixyl 500 et Matrixyl 3000 ; le lycopène ; les sels de manganèse et de magnésium, en particulier les gluconates ; et leurs mélanges.

Une composition de l'invention peut mettre en oeuvre notamment du sodium hyaluronate à titre d'agent anti-âge. Avantageusement, les compositions selon l'invention contiennent de l'acide n-octanoyl-5-salicylique.

L'actif anti-rides additionnel peut représenter au moins 0,05 %, de préférence au moins 0,5 % et plus préférentiellement au moins 1 % en poids par rapport au poids total de la composition.

### Agents hydratants

Comme agents humectants ou hydratants, on peut citer notamment l'urée et ses dérivés notamment l'Hydrovance® commercialisée par National Starch, des monosaccharides comme le mannose, l'acide hyaluronique, les AHA, les BHA, des homopolymères d'acide acrylique comme le Lipidure-HM® de NOF corporation, le beta-glucan et en particulier le sodium carboxymethyl beta-glucane de Mibelle-AG-Biochemistry ; un polyoxybutylène polyoxyéthylène polyoxypropylène glycérol comme le WILBRIDE S-753L® de NOF corporation, une huile de rosier muscat commercialisée par Nestlé ; des sphères de collagène et de chondroitine sulfate d'origine marine (Ateocollagen) commercialisées par la société Engelhard Lyon sous la dénomination sphères de comblement marines ; des sphères d'acide hyaluronique telles que celles commercialisées par la société Engelhard Lyon. Les compositions selon l'invention contiendront en particulier des céramides, et notamment un céramide 5, du glycérol et/ou du karité.

### Filtres UV

A titre d'illustration des filtres UV et de façon non limitative, on peut citer les familles suivantes :
les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40® disponible chez B.A.S.F.) ; les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène di-camphre sulfonique , et préférentiellement le 4-méthylbenzylidène camphre (Eusolex 6300® disponible chez Merck) ; les benzimidazoles, en particulier le benzimidazilate (Neo Heliopan AP® disponible chez Haarmann et Reimer), ou l'acide phénylbenzimidazole sulfonique (Eusolex 232® disponible chez Merck) ; les benzotriazoles, en particulier le drométrizole trisiloxane, ou le méthylène bis-benzotriazolyltétraméthylbutylphénol (Tinosorb M® disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35® disponible chez B.A.S.F.), l'octylméthoxycinnamate (Parsol MCX® disponible chez Hoffmann La Roche), ou l'octocrylène (Uvinul 539® disponible chez B.A.S.F.) ; les dibenzoylméthanes, en particulier le butyl méthoxydibenzoylméthane (Parsol 1789®) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB® disponible chez 3V Sigma), l'éthylhexyltriazone (Uvinul T150® disponible chez B.A.S.F.), ou l'éthyl PABA (benzocaïne) ; le Mexoryl® ; les salicylates, en particulier le salicyclate de dipropylèneglycol, le salicylate d'éthylhexyle, l'homosalate, ou le TEA salicylate ; les triazines, en particulier l'anisotriazine (Tinosorb S® disponible chez Ciba) ; le drometrizole trisiloxane, l'oxyde de zinc, le dioxyde de titane, l'oxyde de zinc, de fer, de zirconium, de cerium enrobés ou non.

De préférence, on utilise les cinnamates, les salicylates et leurs mélanges.

La quantité de filtres dépend de l'utilisation finale souhaitée. Elle peut aller par exemple de 1 à 30 % en poids et mieux de 2 à 210 % en poids par rapport au poids total de la composition le contenant.

### Agents dépigmentants

Comme agents dépigmentants, on peut citer les céramides, la vitamine C et ses dérivés et notamment la vit CG, CP et 3-O éthyl vitamine C, l'alpha et la béta arbutine, l'acide férulique, l'acide kojique, le résorcinol et ses dérivés, le D calcium panthéteine sulfonate, l'acide lipoique, l'acide ellagique, la vitamine B3, le phényléthyl résorcinol comme le Symwhite 377® de la société Symrise, une eau de fruit de kiwi (Actinidia chinensis) commercialisée par Gattefosse, un extrait de racine de Paeonia suffructicosa tel que celui commercialisé par la société Ichimaru Pharcos sous la dénomination Botanpi Liquid B® , un extrait de sucre brun (Saccharum officinarum), tel que l'extrait de melasse commercialisé par la société Taiyo Kagaku sous la dénomination Molasses Liquid, un mélange d'acide undecylenique et phénylalanine undecylenoyl, tel que le Sepiwhite MSH® de Seppic.

### Agents antioxydants

Comme agent antioxydant préféré, on peut plus particulièrement citer le tocophérol et ses esters, en particulier l'acétate de tocophérol ; l'EDTA, l'acide ascorbique et ses dérivés, en particulier l'ascorbyl magnésium phosphate et l'ascorbyl glucoside ; les chélatants, tels que le BHT, le BHA, le N,N'-bis(3,4,5-triméthoxybenzyl) éthylenediamine et ses sels , et leurs mélanges.

Plus préférentiellement, on utilisera le tocopherol et ses esters.

Ainsi, une composition conforme à l'invention peut comprendre un agent antioxydant dans une teneur allant de 0,001 % à 10 % et préférentiellement de 0,01 % à 5 % en poids par rapport au poids total de la composition.

### Agents dermorelaxants ou dermodécontractants

Comme agents dermorelaxants préférés, on peut citer tout particulièrement le gluconate de manganèse, le wild yam, la criste marine, la glycine et l'alvérine.

Une composition conforme à l'invention peut comprendre un agent dermorelaxant dans une teneur allant de 0,0001 % à 5 % et préférentiellement de 0,001 % à 3 % en poids par rapport au poids total de la composition.

### Actifs stimulant la synthèse de macromolécules dermiques et/ou épidermiques et/ou empêchant leur dégradation

Comme actifs préférés stimulant la synthèse de macromolécules dermiques et/ou épidermiques et/ou empêchant leur dégradation, on peut citer : les peptides extraits de végétaux, tels que l'hydrolysat de soja commercialisé par la société BASF Beauty Care Solutions sous la dénomination commerciale Phytokine® l'extrait de malt tel que commercialisé sous la dénomination Collalift® par la société Engelhard Lyon ; les peptides de riz tel que le Nutripeptide® de SILAB, ou encore un extrait de peptides de riz tel que la Colhibin® de Pentapharm, le méthylsilanol mannuronate tel que l'Algisium C@ commercialisé par Exsymol ; un extrait de vaccinium myrtillus tel que ceux décrits dans la demande FR-A-2 814 950 ; l'extrait de lupin commercialisé par la société SILAB sous la dénomination commerciale Structurine®, et leurs mélanges.

Une composition conforme à la présente invention peut comprendre un actif stimulant la synthèse des macromolécules dans une teneur allant de 0,001 % à 10 % et préférentiellement de 0,01 % à 5 % en poids par rapport au poids total de la composition. La composition selon l'invention pourra encore contenir de l'eau de Vichy, riche en minéraux, pour son action fortifiante, anti-radicalaire, et apaisante.

Selon un mode de réalisation avantageux, la composition selon l'invention comprend au moins un actif additionnel choisi parmi l'acide n-octanoyl-5-salicylique, le mannuronate de méthyl silane-triol, et les polysaccharides riches en fucose (Biosaccharide gum-1 ou Fucogel®).

### Milieu physiologiquement acceptable

Une composition contenant une association selon l'invention peut être destinée à une application cosmétique ou pharmaceutique, particulièrement dermatologique. De préférence cette composition selon l'invention est destinée à une application cosmétique. Elle est formulée dans un milieu physiologiquement acceptable.

Le milieu physiologiquement acceptable est préférentiellement un milieu cosmétiquement ou dermatologiquement acceptable, c'est-à-dire sans odeur, couleur ou aspect désagréable, et qui ne génère pas de picotement, tiraillement ou rougeur inacceptable pour l'utilisateur.

Par milieu physiologiquement acceptable, on comprend un milieu compatible avec les matières kératiniques d'êtres humains, en l'occurrence la peau.

La composition selon l'invention peut se présenter sous toutes les formes galéniques normalement utilisées dans les domaines cosmétique et dermatologique.

Elle peut être notamment sous forme d'une solution aqueuse, hydroalcoolique, éventuellement gélifiée, d'une dispersion du type lotion éventuellement biphasée, d'une émulsion huile-dans-eau ou eau-dans-huile ou multiple, d'un gel aqueux, d'une dispersion d'huiles dans une phase aqueuse, notamment à l'aide de sphérules, ces sphérules pouvant être des particules polymériques ou mieux, des vésicules lipidiques de type ionique et/ou non-ionique, ou bien encore sous la forme d'une poudre, d'un sérum, d'une pâte ou d'un bâtonnet souple.

Ainsi, la composition peut comprendre tous les constituants usuellement employés dans l'application envisagée.

On peut notamment citer l'eau, les solvants, les huiles d'origine minérale, animale et/ou végétale notamment telles que détaillées ci-après, les cires telles que décrites ci-après, en particulier les pigments, les charges, les tensioactifs, les gélifiants, les conservateurs. Une composition de l'invention peut avantageusement présenter un toucher ferme et compact à la prise. Elle peut être épaisse à l'application et se transformer ensuite, fondre et libérer de la fraîcheur.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

Les compositions selon l'invention contiennent avantageusement au moins une phase grasse liquide.

Avantageusement, les compositions selon l'invention peuvent se présenter sous la forme d'émulsions.

Les compositions selon l'invention peuvent avantageusement se présenter sous forme d'une émulsion obtenue par dispersion d'une phase aqueuse dans une phase grasse (E/H) ou d'une phase grasse dans une phase aqueuse (H/E), de consistance liquide ou semi-liquide du type lait, ou de consistance molle, semi-solide ou solide du type crème ou gel, ou encore d'émulsion multiple (E/H/E ou H/E/H). Ces compositions sont préparées selon les méthodes usuelles.

Les compositions de ce type peuvent avoir la forme d'un produit de soin ou de maquillage du visage et/ou du corps, et être conditionnée par exemple sous forme de crème en pot ou de fluide en tube ou en flacon pompe.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsionnants sont généralement présents dans la composition, en une proportion pouvant aller par exemple de 0,3 à 30 % en poids, et de préférence de 0,5 à 20 % en poids par rapport au poids total de la composition.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C8-C24, et leurs dérivés oxyalkylénés ; les esters de polyéthylène glycol et d'acide gras en C8-C24, et leurs dérivés oxyalkylénés ; les esters de sorbitol et d'acide gras en C8-C24, et leurs dérivés oxyalkylénés ; les esters de sucre (sucrose, glucose, alkylglucose) et d'acide gras en C8-C24, et leurs dérivés oxyalkylénés ; les éthers d'alcools gras ; les éthers de sucre et d'alcools gras en C8-C24, et leurs mélanges.

Comme exemples d'huiles utilisables dans la composition selon l'invention, on peut citer :
- les huiles hydrocarbonées d'origine animale, telles que le perhydrosqualène,
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité,
- les esters et les éthers de synthèse, notamment d'acides gras, comme les huiles de formules R1COOR2 et R1OR2 dans laquelle R1 représente le reste d'un acide gras comportant de 8 à 29 atomes de carbone, et R2 représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle,

- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam®,
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote,
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique,
- les huiles fluorées partiellement hydrocarbonées et/ou siliconées comme celles décrites dans le document JP-A-2-295912,
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes, et
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool. Les autres corps gras pouvant être présents dans la phase huileuse sont par exemple les cires et les acides gras comportant de 8 à 30 atomes de carbone, comme l'acide stéarique, l'acide laurique, l'acide palmitique et l'acide oléique.

A titre de cires pouvant être utilisées selon l'invention, on peut citer les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre, les cires minérales, par exemple de paraffine, de vaseline, de lignite ou les cires microcristallines ou les ozokérites, les cires synthétiques parmi lesquelles les cires de polyéthylène, de polytétrafluoroéthylène et les cires obtenues par synthèse de Fisher-Tropsch ou encore les cires de silicone, les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C20-C40 vendu sous la dénomination commerciale « KESTER WAX K82H » par la société KOSTER KEUNEN.

Ces corps gras peuvent être choisis de manière variée par l'homme du métier afin de préparer une composition ayant les propriétés, par exemple de consistance ou de texture, souhaitées.

Les compositions selon l'invention peuvent comprendre une huile volatile.

Par « huile volatile », on entend au sens de l'invention une huile susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa (10-3 à 300 mm de Hg), en particulier allant de 1,3 Pa à 13 000 Pa (0,01 à 100 mm de Hg), et plus particulièrement allant de 1 ,3 Pa à 1300 Pa (0,01 à 10 mm de Hg).

On peut citer comme huiles volatiles entre autres, les silicones cycliques ou linéaires renfermant de 2 à 6 atomes de silicium, telles que le cyclohexasiloxane, le dodecamethylpentasiloxane, le decamethyltetrasiloxane, le butyltrisiloxane et l'éthyltrisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de « PF 5050® » et « PF 5060® » par la Société 3M et les dérivés de perfluoromorpholine, tels que la 4-trifluorométhyl perfluoromorpholine vendue sous la dénomination « PF 5052® » par la Société 3M.

La quantité de phase huileuse présente dans les compositions selon l'invention peut aller par exemple de 0,01 à 50 % en poids et de préférence de 0,1 à 30 % en poids par rapport au poids total de la composition.

La composition selon l'invention pourra aussi contenir des élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Les compositions selon l'invention peuvent en outre comprendre au moins une matière colorante choisie par exemple parmi les pigments, les nacres, les colorants, les matériaux à effet et leurs mélanges.

Ces matières colorantes peuvent être présentes en une teneur allant de 0,01 % à 50 % en poids, de préférence de 0,01 % à 30 % par rapport au poids total de la composition. Les compositions selon l'invention peuvent également comprendre une charge notamment en une teneur allant de 0,01 % à 50 % en poids, par rapport au poids total de la composition de préférence allant de 0,01 % à 30 % en poids. Ces charges peuvent être minérales ou organiques de toute forme, plaquettaires, sphériques ou oblongues, quelle que soit la forme cristallographique (par exemple feuillet, cubique, hexagonale, orthorombique, ou amorphe). On peut citer la silice, le talc, le mica, le kaolin, la lauroyl-lysine, l'amidon, le nitrure de bore, les poudres de PTFE, les poudres de PMMA, les poudres de résine de methyl silsesquioxane (comme le Tospearl 145A de GE Silicone) , les particules hémisphérique creuse de résine de silicone (comme les NLK 500, NLK 506 et NLK 510 de Takemoto Oil and Fat), le sulfate de Baryum, le carbonate de calcium précipité, le carbonate et l'hydro-carbonate de magnésium, l'hydroxyapatite, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut en outre contenir divers adjuvants couramment utilisés dans le domaine cosmétique, tels que des séquestrants ; des parfums ; et des épaississants et gélifiants. Les quantités de ces différents adjuvants et leur nature seront choisies de manière à ne pas nuire aux propriétés de la composition.

L'invention a en outre pour objet un procédé de traitement cosmétique pour prévenir et/ou retarder et/ou limiter les signes du vieillissement de la peau et/ou de ses annexes, dans applique topiquement au moins une composition ou une association telles que définies précédemment.

Par signes cutanés du vieillissement on entend toutes modifications de l'aspect extérieur de la peau dues au vieillissement qu'il soit chronobiologique et/ou photo-induit, comme par exemple les rides et ridules, la peau flétrie, la peau molle, la peau amincie, la peau terne et sans éclat, le manque d'élasticité et/ou de tonus de la peau, mais également toutes modifications internes de la peau qui ne se traduisent pas systématiquement par un aspect extérieur modifié, comme par exemple toutes dégradations internes de la peau, particulièrement des fibres de collagène, consécutives à une exposition aux rayonnements ultra-violets, qui peuvent avoir comme conséquence l'amincissement du derme.

Les compositions selon l'invention sont utiles pour lutter contre les rides, notamment les rides disposées radialement autour de la bouche et/ou des yeux, en particulier les rides de la patte d'oie, et/ou situées au niveau du front, en particulier la ride dite du lion, située au niveau de la glabelle, dans l'espace inter-sourcillier, et/ou disposées horizontalement sur le front.

Le procédé de traitement cosmétique de la peau, notamment d'une peau ridée, sera en particulier adapté à la peau du visage et/ou du cou, et comprend l'application topique sur ladite peau d'une composition comprenant, dans un milieu physiologiquement acceptable l'association d'au moins un extrait de bactérie filamenteuse non photosynthétique et d'au moins un extrait de Cassia alata, en particulier de feuilles de Cassia alata.

La composition ou l'association sont en particulier destinées à lutter contre les dégradations du collagène. L'invention a ainsi pour objet une association d'au moins un extrait d'une bactérie filamenteuse non photosynthétique et d'au moins un extrait de cassia alata ou une composition, notamment cosmétique la contenant telles que définies plus haut, pour améliorer la structure du derme et/ou augmenter sa densité (caractérisée par le rapport derme supérieur/derme inférieur).

L'invention a également pour objet l'utilisation d'une composition ou d'une association telles que définies dans ce qui précède, pour inhiber l'expression et/ou la synthèse et/ou l'activité des protéases de la matrice extracellulaire, particulièrement des métalloprotéinases et encore plus particulièrement de la métalloprotéinase de type 1.

L'invention a également pour objet un procédé de traitement cosmétique pour prévenir et/ou traiter les matières kératiniques sèches, les peaux sèches et/ou fragilisées, et/ou pour traiter les peaux sèches hypo-séborrhéiques, et/ou pour stimuler la sébogénèse, et/ou pour le traitement et/ou la prévention des désordres cutanés et/ou de l'unité pilosébacée liés à un défaut d'excrétion et/ou de sécrétion de sébum, et/ou pour prévenir et/ou traiter les fibres kératiniques sèches ou fragilisées, et/ou pour prévenir et/ou réduire les rides liées à un sécheresse cutanée, comprenant l'application topique d'au moins une composition ou une association telles que définies plus haut.

L'invention a encore pour objet un procédé de traitement cosmétique pour favoriser l'amincissement ou l'affinement du corps et de la silhouette ou de certaines parties du corps et de la silhouette, caractérisé par le fait que l'on applique sur la peau une composition ou une association telles que définies plus haut. l'utilisation dans ce cadre des associations ou compositions selon l'invention visera notamment à diminuer la cellulite et l'aspect capitonné de la peau.

L'invention a encore pour objet un procédé cosmétique pour éclaircir la couleur de la peau ou des muqueuses comprenant l'application topique d'au moins une composition ou une association telles que définies dans ce qui précède.

Les procédés de traitement cosmétique selon l'invention sont en particulier efficaces pour diminuer les signes du vieillissement et diminuer l'âge apparent des sujets qui l'appliquent.

Les compositions ou les associations selon l'invention sont ainsi efficaces pour améliorer l'hydratation de la peau et/ou améliorer les propriétés bio-mécaniques de la peau et/ou l'éclat et l'homogénéité du teint et/ou diminuer les rides et les ridules et/ou lisser la peau. La mise en oeuvre de l'invention permet notamment d'améliorer l'élasticité, la tonicité et/ou la fermeté de la peau.

Dans les procédés selon l'invention, l'association d'extrait de bactérie filamenteuse non photosynthétique telle que Vitreoscilla filiformis et d'un extrait de Cassia alata, ou la composition la contenant, seront appliquées de préférence topiquement sur la peau, les muqueuses et/ou les fibres kératiniques. L'application pourra être quotidienne ou biquotidienne. Dans le cas de traitement cosmétique pour retarder ou prévenir les signes du vieillissement, l'application pourra notamment être renouvellée avant, pendant et/ou après une exposition aux rayonnements U.V.

Des résultats pourront être observés dès l'application du traitement cosmétique. Toutefois, le traitement cosmétique selon l'invention pourra être prolongé pendant plusieurs semaines, voire plusieurs mois.

L'association ou la composition la contenant seront appliquées de préférence sur les zones de peau concernées par les désordres contre lesquels on veut lutter. Ces zones sont notamment la peau du visage, du cou des bras et des mains, mais aussi des régions corporelles comme les jambes, les cuisses ou l'abdomen ou toute zone à traiter.

L'invention concerne également l'utilisation d'une association d'au moins un extrait d'une bactérie filamenteuse non photosynthétique et non fructifiante et au moins un extrait de la plante Cassia alata, comme agent pour retarder et/ou prévenir les signes du vieillissement cutané et/ou comme agent pour lutter contre la déshydratation de la peau et/ou pour lutter contre les dégradations de la peau provoquées par les rayonnements U.V.

Il est entendu que les différents modes de réalisation exposés dans ce qui précède peuvent être combinés dans le cadre de l'invention.

D'autres avantages de la présente invention apparaitront à la lecture des exemples qui suivent.

### Exemple 1 : Effet sur l'expression des marqueurs transglutaminase (TGK) et Tissue Inhibitor of Matrix metalloprotease-1 (TIMP1) dans un modèle d'épiderme reconstruit

L'effet *in vitro* de 2 actifs, Vitreoscilla ferment et cassia alata leaf extract, et de leur association a été évalué sur l'expression de marqueurs impliqués dans la différenciation épidermique (TGK) et le remodelage de la matrice extracellulaire (TIMP1). Pour cela, nous avons mis en culture des épidermes reconstruits SkinEthic de 17 jours pendant 24h dans leur milieu de culture, avant de les traiter de manière systémique avec les composés à l'essai ou leur association, et analysé l'expression des gènes codant pour les protéines TGK et TIMP1 grâce à la technique de RT-PCR quantitative.

### Mode opératoire

Les épidermes reconstruits Skinethic ont été mis en culture pendant 24h dans leur milieu de culture (milieu de maintenance), à 37°C et 5 % CO₂. Le milieu est ensuite remplacé par du milieu de maintenance contenant ou non (témoin) les composés à l'essai ou leur association. Les épidermes ont ensuite été incubés pendant 6 et 24 heures. Toutes les conditions expérimentales ont été réalisées en duplicata (n=2).

A la fin des incubations le milieu a été retiré, les épidermes ont été rincés avec du PBS, découpés et congelés à -80°C en présence de Tri-reagent.

Après extraction des ARN totaux, une réaction de reverse transcription de l'ARNm a été réalisée, puis une PCR quantitative.

Les couples de primers qui ont été utilisés dans cette étude permettent l'amplification des fragments spécifiques Transglutaminase 1 (TGM1) et Tissues inhibitor of metalloproteinase 1 precursor (TIMP1).

### Résultats

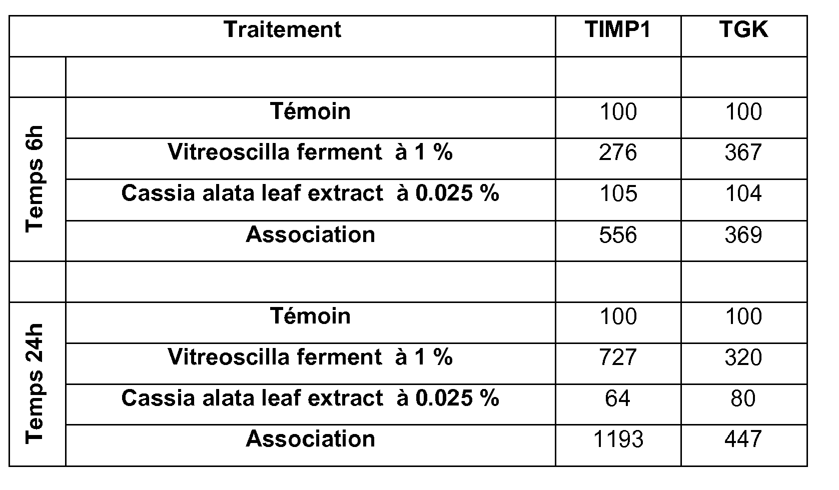

Dans les conditions expérimentales de cette étude, le *Vitreoscilla ferment* appliqué en systémique à 1% induit après 6h d'incubation une nette stimulation de l'expression du gène codant pour les marqueurs TIMP1, et TGK (respectivement + 176 %, et + 267 % par rapport au témoin).

Après 24h d'incubation, l'expression de la TIMP1 stimulée de manière plus importante qu'à 6h (+627 % par rapport au témoin), alors que celles de la TGK reste au même niveau.

Le traitement avec l'actif *Cassia alata leaf extract* appliqué en systémique à 0.025 % pendant 6h est sans effet sur les marqueurs étudiés.

Par contre, après 24h d'incubation, cet actif induit une tendance à diminuer de l'expression du gène codant pour le gène codant pour TIMP1 (-36 % par rapport au témoin). Il est sans effet net sur l'expression de la TGK.

L'association *Vitreoscilla ferment* / *Cassia alata leaf extract* induit après 6h d'incubation une stimulation de l'expression du gène codant pour TIMP1 plus importante que le *Vitreoscilla ferment* testé seul (+456 % versus +176 %). Il y a donc une synergie puisque au lieu de 2,76 X1.03 (soit 2,84) que l'on attend, on constate une induction de x 5,56 De plus, il a les mêmes effets que le Vitreoscilla ferment seule sur l'expression de la TGK (+289 %).

Après 24h d'incubation, cette association induit une stimulation très importante de l'expression de la TIMP1 (+1093% par rapport au témoin) et importante de la TGK (+347 %), tous deux de manière plus importante qu'avec les actifs seuls. Pour le TGK, on constate une induction de 4,47, alors que la simple addition des effets obtenus par chaque actif séparément conduirait à une induction de 2,56. Il y a donc potentialisation de l'effet sur les 2 marqueurs TGK et TIMP après 24 h.

### Conclusion

L'association des actifs *Vitreoscilla ferment* et *Cassia alata leaf extract* permet, avec le temps, d'augmenter l'expression des gènes codant pour les protéines TGK et TIMP1, de manière plus importante que les actifs seuls. Les effets du *Vitreoscilla ferment* semblent donc potentialisés par le *Cassia alata leaf extract* lorsque les épidermes reconstruits sont traités en systémique.

Les résultats obtenus pour cette association d'actifs traduisent donc une stimulation de la différenciation épidermique, et une protection de la matrice extracellulaire vis-à-vis de la dégradation par la MMP1 (matrix metalloproteinase 1)

### Compositions selon l'invention (les quantités sont exprimées en pourcentage en poids)

### Exemple 2: Compositions éclaircissantes

### Sérum éclaircissant

| | |
|---|---|
| HYDROXYDE DE SODIUM PUR | 0,0130 |
| ACIDE ETHYLENE DIAMINE TETRACETIQUE, SEL TETRASODIQUE | 0,10 |
| ACIDE N-OCTANOYL-5 SALICYLIQUE | 0,30 |
| Vitreoscilla filiformis en dispersion aqueuse | 1 |
| PHENOXY-2 ETHANOL | 0,50 |
| OCTYL-2 DODECANOL | 2 |
| ISO-NONANOATE D'ISO-NONYLE | 2 |
| ALCOOL CETYLIQUE PUR BIDISTILLE | 1 |
| ACIDE ELLAGIQUE | 0,50 |
| EXTRAIT DE FEUILLE DE SENNA (OU CASSIA) ALATA | 0,025 |
| PARFUM | 0,07 |
| GOMME DE XANTHANE | 0,10 |
| COPOLYMERE ACRYLAMIDE/ACRYLAMIDO 2-METHYL PROPANE SULFONATE DESODIUM EN EMULSION INVERSE A 40% DANS ISOPARAFFINE/EAU | 1,60 |
| POLY DIMETHYLSILOXANE (VISCOSITE: 5 CST) | 5 |
| 1,3-BUTYLENE GLYCOL | 7 |
| EAU DESIONISEE | qsp 100 |
| GLYCEROL | 5 |
| ACIDES GRAS (ACIDE STEARIQUE MAJORITAIRE) D'ORIGINE VEGETALE | 1,20 |
| MELANGE MONO/DISTEARATE DE GLYCERYLE / STEARATE DE POLYETHYLENE GLYCOL (100 OE) | 1,42 |
| METHYL-GLUCOSE OXYETHYLENE (20 OE) | 3 |
| ALCOOL CETYLIQUE OXYETHYLENE (20 OE) OXYPROPYLENE (5 OP) | 0,10 |
| ACETATE DE DL-ALPHA-TOCOPHERYLE (ACETATE DE VITAMINE E) | 0,50 |

### Sérum éclat:

| | |
|---|---|
| ACIDE ETHYLENE DIAMINE TETRACETIQUE, SEL TETRASODIQUE | 0,05 |
| TRIETHANOLAMINE | 0,40 |
| Vitreoscilla filiformis en dispersion aqueuse | 1 |
| PHENOXY-2 ETHANOL | 0,70 |
| BEURRE DE KARITE PROTEGE | 0,50 |
| TETRA-OCTANOATE DE PENTAERYTHRITYLE | 5 |
| ALCOOL CETYLIQUE PUR BIDISTILLE | 1 |
| VASELINE BLANCHE | 1,50 |
| EXTRAIT DE FEUILLE DE SENNA (OU CASSIA) ALATA | 0,025 |
| PARFUM | 0,20 |
| ISOPARAFFINE (6-8 MOLES D'ISOBUTYLENE) HYDROGENEE | 3 |
| GOMME DE XANTHANE | 0,15 |
| COPOLYMERE ACRYLAMIDE/ACRYLAMIDO 2-METHYL PROPANE SULFONATE DESODIUM EN EMULSION INVERSE A 40% DANS ISOPARAFFINE/EAU | 2 |
| COPOLYMERE ACIDE ACRYLIQUE/METHACRYLATE DE STEARYLE POLYMERISE DANS UN MELANGE ACETATE D'ETHYLE/CYCLOHEXANE | 0,10 |
| CYCLOHEXA DIMETHYLSILOXANE (VISCOSITE: 8 CST) | 2,50 |
| EAU DESIONISEE | qsp 100 |
| GLYCEROL | 3 |
| ACIDES GRAS (ACIDE STEARIQUE MAJORITAIRE) D'ORIGINE VEGETALE | 1,30 |
| MELANGE MONO/DISTEARATE DE GLYCERYLE / STEARATE DE POLYETHYLENE GLYCOL (100 OE) | 1,50 |
| ACIDE GLUCOPYRANOSYL-L-ASCORBIQUE | 2 |

### Exemple 3: compositions anti-âge

### Crème anti-âge

| | |
|---|---|
| ACIDE ETHYLENE DIAMINE TETRACETIQUE, SEL DISODIQUE, 2 H2O | 0,05 |
| MELANGE DE TOCOPHEROLS NATURELS DANS HUILE DE SOJA (50/50) | 0,10 |
| ACIDE N-OCTANOYL-5 SALICYLIQUE | 0,50 |
| TRIETHANOLAMINE | 0,50 |
| Vitreoscilla filiformis en dispersion aqueuse | 2 |
| PHENOXY-2 ETHANOL | 0,70 |
| OCTYL-2 DODECANOL | 3 |
| BEURRE DE KARITE PROTEGE | 3 |
| TETRA-OCTANOATE DE PENTAERYTHRITYLE | 1 |
| MÉLANGE D'AMANDES D'ABRICOT | 1 |
| EXTRAIT DE FEUILLE DE SENNA (OU CASSIA) ALATA | 0,025 |
| ISOPARAFFINE (6-8 MOLES D'ISOBUTYLENE) HYDROGENEE | 1,75 |
| COPOLYMERE ACRYLIQUE SOLUBLE EN MILIEU ALCALIN EN DISPERSION AQUEUSE A 30 % NON STABILISE | 0,50 |
| POLYMERE ACIDE ACRYLIQUE/ACRYLATE D'ALKYLES RÉTICULÉ | 0,07 |
| ACIDE POLY ACRYLAMIDOMETHYL PROPANE SULFONIQUE NEUTRALISE PARTIELLEMENT A L'AMMONIAQUE ET HAUTEMENT RETICULE | 1,90 |
| MELANGE DE POLY DIMETHYLSILOXANE RETICULE ET DE POLY DIMETHYLSILOXANE (6 CST) (24/76) | 5 |
| 1,3-BUTYLENE GLYCOL | 3 |
| EAU DESIONISEE | qsp 100 |
| GLYCEROL | 5 |
| MONO/DISTEARATE DE GLYCERYLE | 0,05 |
| PROVITAMINE B5 : D-PANTHENOL | 0,30 |

On appliquera la crème sur la peau en particulier du visage, du cou et/ou des mains, le matin et/ou le soir

### Lotion anti-âge:

| | |
|---|---|
| ACIDE ETHYLENE DIAMINE TETRACETIQUE, SEL TETRASODIQUE | 0,05 |
| ACIDE N-OCTANOYL-5 SALICYLIQUE | 0,30 |
| TRIETHANOLAMINE | 0,20 |
| Vitreoscilla filiformis en dispersion aqueuse | 2 |
| ADENOSINE | 0,04 |
| PHENOXY-2 ETHANOL | 0,30 |
| EXTRAIT DE FEUILLE DE SENNA (OU CASSIA) ALATA | 0,00250 |
| PARFUM | 0,0050 |
| HYALURONATE DE SODIUM (PM : 1.100.000) EN POUDRE | 0,05 |
| ALCOOL ETHYLIQUE ABSOLU DENATURE | 7,50 |
| EAU DESIONISEE | qsp 100 |
| GLYCEROL | 5 |
| METHYL GLUCOSIDE OXYPROPOXYLE (10 OP) | 0,80 |

### Exemple 4:

### Crème de protection solaire

| | |
|---|---|
| ACIDE ETHYLENE DIAMINE TETRACETIQUE, SEL DISODIQUE, 2 H2O | 0,05 |
| MELANGE DE TOCOPHEROLS NATURELS DANS HUILE DE SOJA (50/50) | 0,10 |
| TRIETHANOLAMINE | 0,80 |
| Vitreoscilla filiformis en dispersion aqueuse | 2 |
| PHENOXY-2 ETHANOL | 0,50 |
| HUILE D'AMANDES D'ABRICOT DESODORISEE RAFFINEE NON STABILISEE | 0,50 |
| EXTRAIT DE FEUILLE DE SENNA (OU CASSIA) ALATA | 0,025 |
| TRI-ANILINO-2,4,6 P-(CARBO ETHYL-2 HEXYL OXY-1') TRIAZINE-1,3,5 | 2 |
| ACIDE B-B'CAMPHOSULFONIQUE [1-4 DIVINYLBENZENE] EN SOLUTION AQUEUSE | 3 |
| FILTRE BENZOTRIAZOLE DERIVE DE L'HEPTAMETHYLE HYDROGENO-TRISILOXANE | 4 |
| P-METHOXY-4 CINNAMATE D'ETHYL-2 HEXYLE STABILISE (BHT 0,1 %) | 7,50 |
| DERIVE TRIAZINIQUE (BEMT) = (2,4-BIS((4-(2-ETHYL-HEXYLOXY)-2-HYDROXY)-PHENYL)-6-(4-METHOXYPHENYL)-(1,3,5)-TRIAZINE) | 1 |
| PARFUM | 0,20 |
| ACIDE POLY ACRYLAMIDOMETHYL PROPANE SULFONIQUE NEUTRALISE PARTIELLEMENT A L'AMMONIAQUE ET HAUTEMENT RETICULE | 1 |
| ALCOOL ETHYLIQUE ABSOLU DENATURE | 10 |
| EAU DESIONISEE | qsp 100 |
| BENZOATE D'ALCOOLS C12/C15 | 5 |
| GLYCEROL | 3 |
| ALCOOL ISOCETYLIQUE OXYETHYLENE (20 OE) | 0,10 |

La crème sera appliquée sur l'ensemble du corps avant et/ou pendant une exposition aux rayonnements solaires.

### Exemple 5

### Gel anti-cellulite

| | |
|---|---|
| CAFEINE (1,3,7-TRIMETHYL XANTHINE) ANHYDRE | 3 |
| TRIETHANOLAMINE | 1,68 |
| Vitreoscilla filiformis en dispersion aqueuse | 1 |
| ACIDE SALICYLIQUE EN POUDRE | 0,20 |
| FRACTION LIQUIDE DE BEURRE DE KARITE PROTEGEE | 1 |
| EXTRAIT AQUEUX SEC DE GINKGO BILOBA NON STABILISE | 0,10 |
| EXTRAIT DE FEUILLE DE SENNA (OU CASSIA) ALATA | 0,025 |
| PARFUM | 0,30 |
| POLYMERE CARBOXYVINYLIQUE SYNTHETISE DANS LE MELANGE ACETATE D'ETHYLE/CYCLOHEXANE | 0,50 |
| ACIDE POLY ACRYLAMIDOMETHYL PROPANE SULFONIQUE NEUTRALISE PARTIELLEMENT A L'AMMONIAQUE ET HAUTEMENT RETICULE | 0,40 |
| POLY DIMETHYLSILOXANE (VISCOSITE: 10 CST) | 8 |
| ALCOOL ETHYLIQUE ABSOLU DENATURE | 20 |
| EAU DESIONISEE | qsp 100 |
| GLYCEROL | 3 |

Le gel est appliqué sur le corps, en particulier sur les hanches, les cuisses et le ventre, matin et/ou soir.

## Revendications

1. Composition **caractérisée en ce qu'**elle contient dans un milieu physiologiquement acceptable l'association d'au moins un extrait d'une bactérie filamenteuse non photosynthétique et non fructifiante et au moins un extrait de la plante Cassia alata.

2. Composition selon la revendication 1 **caractérisée en ce que** ladite bactérie est *Vitreoscilla filiformis.*

3. Composition selon l'une des revendications 1 ou 2, **caractérisée en ce que** l'extrait de Cassia alata est un extrait de feuille de Cassia alata.

4. Composition selon l'une des revendications 1 à 3, **caractérisée en ce que** l'extrait de bactérie filamenteuse non photosynthétique est présent à une concentration de 0,1 à 10% en poids par rapport au poids total de la composition.

5. Composition selon l'une des revendications 1 à 4, **caractérisée en ce que** l'extrait de Cassia alata est présent à une concentration de 0,001% à 1%, de préférence de 0,025 à 0,5 % en poids par rapport au poids total de la composition.

6. Composition selon l'une au moins des revendications précédentes, **caractérisé en ce que** l'association de l'extrait de bactérie filamenteuse et de l'extrait de Cassia alata présente une activité synergique sur l'augmentation de la synthèse de la transglutaminase K et/ou l'augmentation de synthèse de la TIMP1.

7. Composition selon l'une au moins des revendications 1 à 6, **caractérisée en ce qu'**il s'agit d'une composition cosmétique et/ou dermatologique, contenant en outre des excipients adaptés à une application topique sur la peau et/ou les muqueuses et/ou le cuir chevelu.

8. Composition selon l'une des revendications 1 à 7, **caractérisé en ce qu'**elle contient au moins un autre actif choisi parmi les agents anti-âge, les dépigmentants, les filtres U.V. et les agents hydratants.

9. Association synergique d'au moins un extrait de Vitreoscilla filiformis et d'au moins un extrait de Cassia alata, présentant une synergie d'activité sur l'augmentation de la synthèse de la transglutaminase K et/ou augmentation de synthèse de la TIMP1 par les cellules de la peau.

10. Procédé de traitement cosmétique pour prévenir et/ou retarder et/ou limiter les signes du vieillissement de la peau et/ou de ses annexes, **caractérisé en ce qu'**on applique topiquement au moins une composition selon l'une des revendications 1 à 8 ou une association selon la revendication 9.

11. Procédé cosmétique pour prévenir et/ou traiter les matières kératiniques sèches, les peaux sèches et/ou fragilisées, et/ou pour traiter les peaux sèches hypo-séborrhéiques, et/ou pour stimuler la sébogénèse, et/ou pour le traitement et/ou la prévention des désordres cutanés et/ou de l'unité pilosébacée liés à un défaut d'excrétion et/ou de sécrétion de sébum, et/ou pour prévenir et/ou traiter les fibres kératiniques sèches ou fragilisées, et/ou pour prévenir et/ou réduire les rides liées à un sécheresse cutanée, comprenant l'application topique d'au moins une composition selon l'une des revendications 1 à 8 ou une association selon la revendication 9.

12. Procédé cosmétique pour favoriser l'amincissement ou l'affinement du corps et de la silhouette ou de certaines parties du corps et de la silhouette, **caractérisé par le fait que** l'on applique sur la peau une composition selon l'une des revendications 1 à 8, ou une association selon la revendication 9.

13. Procédé cosmétique pour éclaircir la couleur de la peau ou des muqueuses comprenant l'application topique d'au moins une composition selon l'une des revendications 1 à 8 ou d'une association selon la revendication 9.
